(19) Europäisches Patentamt / European Patent Office / Office européen des brevets

(11) **EP 2 508 910 B1**

(12) **EUROPEAN PATENT SPECIFICATION**

(45) Date of publication and mention
of the grant of the patent:
**19.08.2020 Bulletin 2020/34**

(51) Int Cl.:
*G01R 33/54* (2006.01)   *G01R 33/48* (2006.01)
*G01R 33/56* (2006.01)   *A61B 5/055* (2006.01)

(21) Application number: **12160608.1**

(22) Date of filing: **21.03.2012**

(54) **Magnetic resonance imaging system and process**

Magnetresonanzbildgebungssystem und -verfahren

Système d'imagerie par résonance magnétique et procédé

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **22.03.2011 US 201113064376
24.02.2012 JP 2012038573**

(43) Date of publication of application:
**10.10.2012 Bulletin 2012/41**

(73) Proprietor: **Toshiba Medical Systems Corporation
Otawara-shi, Tochigi-ken 324-0036 (JP)**

(72) Inventor: **Wheaton, Andrew J.
324-8550, Tochigi Otawara-shi (JP)**

(74) Representative: **Moreland, David et al
Marks & Clerk LLP
Aurora
120 Bothwell Street
Glasgow G2 7JS (GB)**

(56) References cited:
**JP-A- 2010 162 096     US-A1- 2007 285 090**

**Description**

FIELD

The subject matter below relates generally to magnetic resonance imaging (MRI) processes and apparatus. Preferably, the MRI processes and apparatus described below involve enhancements to separation of nuclear magnetic resonance (NMR) signals emanating from different NMR species (e.g., water molecules and fat molecules).

BACKGROUND

Conventionally, Dixon-based techniques are known as techniques concerned with MRI system for obtaining an image in which water or fat is selectively suppressed. The Dixon-based techniques are methods for generating an water image and an fat image respectively based on MR signals emanating from protons, using the phenomenon that resonant frequencies are slightly different between water and fat.

JP 2010/162096 discloses an apparatus and method according to the respective preambles of the independent claims.

US 2007/285090 discloses a method of using phase cycling in a steady-state imaging techniques.

According to an aspect of the present invention there is provided a magnetic resonance imaging system according to claim 1.

According to another aspect of the present invention there is provided a magnetic resonance imaging process according to claim 6.

BRIEF DESCRIPTION OF THE DRAWINGS

FIG. 1 is a high-level schematic block diagram of an exemplary MRI system embodiment using an enhanced NMR species separation (e.g., water and fat) preparatory pulse sequence (i.e., prior to a main MRI data acquisition sequence);

FIG. 2 is an abbreviated schematic sequence diagram for an exemplary water/fat opposed-phase (WFOP) preparatory sequence followed by a conventional FSE (fast spin echo) MRI pulse sequence;

FIG. 3 includes three comparative MR images of brain tissue;

FIG. 4 is a normalized bar graph comparison of fat, white matter and gray matter in three comparative MR images;

FIG. 5 depicts NMR signal intensities in an exemplary embodiment using a phase-cycling preparatory sequence scheme followed by data combination from multiple such acquisitions (using sum of squares (SSQ) in this example) to create a resultant final image with full signal across an entire spectrum of off-resonance NMR signals;

FIG. 6 depicts four NMR signal acquisitions in another phase-cycling scheme to provide four input images for data combinations;

FIG. 7 provides three comparative MR images demonstrating incorrect fat/water assignment near signal polarity crossings; and

FIG. 8 provides exemplary MR images for an example of fat/water image

DETAILED DESCRIPTION

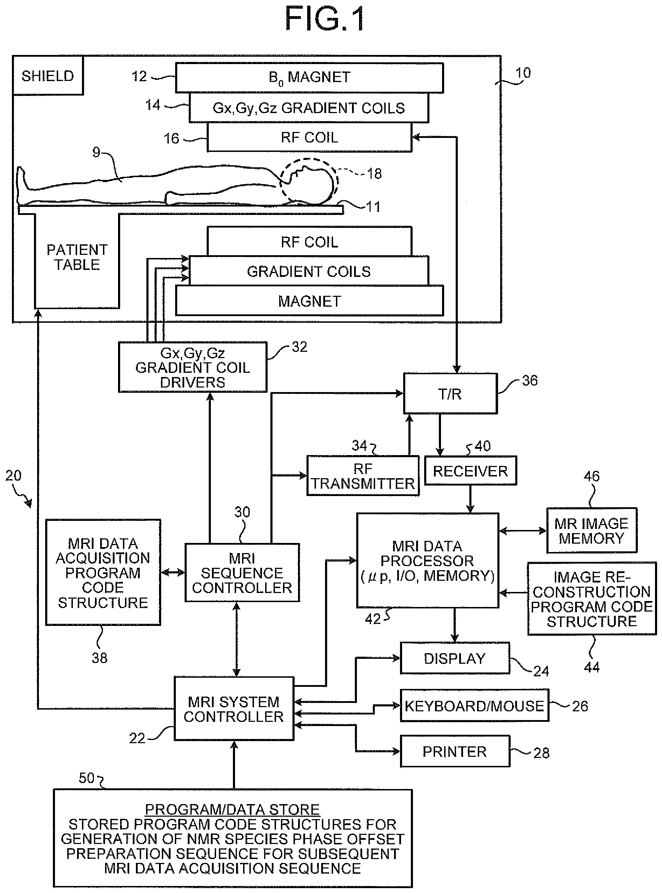

The MRI system shown in FIG. 1 includes a gantry 10 (shown in schematic cross-section) and various related system components 20 interfaced therewith. At least the gantry 10 is typically located in a shielded room. One MRI system geometry depicted in FIG. 1 includes a substantially coaxial cylindrical arrangement of the static field B0 magnet 12, a $G_x$, $G_y$ and $G_z$ gradient coil set 14 and an RF coil assembly 16. Along the horizontal axis of this cylindrical array of elements is an imaging volume 18 shown as substantially encompassing the head of a patient 9 supported by a patient table 11.

An MRI system controller 22 has input/output ports connected to display 24, keyboard/mouse 26 and printer 28. As will be appreciated, the display 24 may be of the touch-screen variety so that it provides control inputs as well.

The MRI system controller 22 interfaces with MRI sequence controller 30 which, in turn, controls the $G_x$, $G_y$ and $G_z$ gradient coil drivers 32, as well as the RF transmitter 34 and the transmit/receive switch 36 (if the same RF coil is used for both transmission and reception). The MRI sequence controller 30 also has access to suitable program code structure 38 for implementing MRI data acquisition sequences already available in the repertoire of the MRI sequence controller 30 using operator and/or system inputs defining particular sequence parameters.

The MRI system 20 includes an RF receiver 40 providing input to data processor 42 so as to create processed image data to display 24. The MRI data processor 42 is also configured for access to image reconstruction program code structure 44 and to MR image memory 46 (e.g., for storing MR image data derived from processing in accordance with the exemplary embodiments and the image reconstruction program code structure 44).

Also illustrated in FIG. 1 is a generalized depiction of an MRI system program/data store 50 where stored program code structures (e.g., for generation of an enhanced preparatory sequence such as a water/fat opposed phase sequence using a binomial RF chemical shift selective preparation pulse) are stored in computer-readable storage media accessible to the various data processing components of the MRI system. As those in the art will appreciate, the program store 50 may be segmented and directly connected, at least in part, to different ones of the system 20 processing computers having most immediate need for such stored program code structures in their normal operation (i.e., rather than being commonly stored and connected directly to the MRI system controller 22).

Indeed, as those in the art will appreciate, the FIG. 1 depiction is a very high-level simplified diagram of a typical MRI system with some modifications so as to practice exemplary embodiments to be described hereinbelow. The system components can be divided into different logical collections of "boxes" that typically comprise numerous digital signal processors (DSP), microprocessors, special purpose processing circuits (e.g., for fast A/D conversions, fast Fourier transforming, array processing, etc.). Each of those processors is typically a clocked "state machine" wherein the physical data processing circuits progress from one physical state to another upon the occurrence of each clock cycle (or predetermined number of clock cycles).

Not only does the physical state of processing circuits (e.g., CPUs, registers, buffers, arithmetic units, etc.) progressively change from one clock cycle to another during the course of operation, the physical state of associated data storage media (e.g., bit storage sites in magnetic storage media) is transformed from one state to another during operation of such a system. For example, at the conclusion of an MR-imaging reconstruction process, an array of computer-readable accessible data value storage sites (e.g., multi-digit binary representations of pixel values) in physical storage media will be transformed from some prior state (e.g., all uniform "zero" values or all "one" values) to a new state wherein the physical states at the physical sites of such an array (e.g., of pixel values) vary between minimum and maximum values to represent real world physical events and conditions (e.g., the tissues of a patient over an imaged volume space). As those in the art will appreciate, such arrays of stored data values represent and also constitute a physical structure - as does a particular structure of computer control program codes that, when sequentially loaded into instruction registers and executed by one or more CPUs of the MRI system 20, cause a particular sequence of operational states to occur and be transitioned through within the MRI system.

The exemplary embodiments described below provide improved ways to acquire and/or process MRI data acquisitions and/or to generate and display MR images.

Standard Dixon-based techniques have been used for fat suppression for over twenty years. In the spin echo (SE) form of Dixon, the RF refocus pulse is shifted in time so that fat and water achieve a desired relative phase at the TE (time to echo), preferably $\pi$ radians out-of-phase. The spin echo (SE) form of Dixon can be extended to fast spin echo (FSE). In the FSE version, the Dixon effect is achieved by time shifting corresponding to the desired out-of-phase time either the position of the first RF refocus pulse or the position of the read echo time ($k_x$=0) within the echo signal. The RF refocus pulse shift approach has the primary disadvantage of alternating the $k_x$ position of the RF echo in each view, resulting in classic N/2 ghosting. The other approach of shifting the position of the read echo within the echo space either extends inter-echo spacing by a full cycle time (4.6 ms at 1.5 T and 2.3 ms at 3 T) or forces asymmetric echo acquisition.

In Dixon-SE and Dixon-FSE, the RF echo and read echo ($k_x$ = 0) are not acquired at the same time. Thus, there is some background phase accrual during this delay. To allow water/fat separation, this background phase must be removed with an estimation of the field map. The estimation of the background phase is non-trivial. As a further drawback to conventional Dixon-FSE with echo spacing extension, long echo spacings are known to impair robustness of the field map estimation.

If multiple Dixon-FSE images are acquired for water/fat separation, the read echo is acquired at a different temporal position within the echo spacing in each image. With this arrangement, each echo is acquired with slightly different phase due to time-dependent sources of phase (i.e., eddy currents from preceding gradients). Therefore, special considerations must be taken to address this phase which, unlike the background phase that can be accounted for, is non-uniform across the data sets.

So-called "binomial" NMR RF excitation pulses have previously been used for spectral-selective (e.g., water) excitation. For example, binomial pulses having plural RF nutation components in nutation angle ratios such as 1:1, 1:2:1, 1:3:3:1, etc., have been used to selectively nutate NMR water-related magnetization into a transverse plane, while leaving magnetization emanating from fat molecules in the longitudinal axis aligned with the static magnetic field $B_0$, thus creating a fat-suppressed image.

According to the invention as described below, a magnetic resonance imaging system includes a sequence executing unit and an image generating unit. The sequence executing unit executes a prep applying portion including a first prepulse for exciting magnetization vectors of a plurality of NMR species having different resonant frequencies as transverse magnetizations and a second prepulse for nutating back the transverse magnetizations into longitudinal magnetization, the second prepulse being applied after a phase evolution time by which a predetermined phase difference is evolved between a subject NMR species and the other NMR species of the plurality of NMR species after the first prepulse, and execute a data acquisition sequence after the prep applying portion. The image generating unit generates a separation image of a subject NMR species based on the data acquired by the data acquisition sequence. The sequence executing unit is implemented in, for example, the MRI sequence controller 30 shown in FIG.1, and the image generating unit is implemented in, for example, the MRI data processor 42.

The invention provides a preparatory NMR sequence using a binomial radio frequency (RF) pulse having at least two independently phased RF flip angle components, the components being spaced in the time domain by $\tau$ to provide a respectively corresponding evolved phase difference $\Delta\theta$ between predetermined NMR species which have different NMR frequencies. Such binomial RF pulses are then followed by at least one magnetic gradient spoiler pulse before a conventional main MRI data acquisition sequence is effected. The MRI data acquired during such conventional sequence may then be used conventionally to generate and display an image of the patient's tissue based, at least in part, on the acquired MRI data.

In a particularly preferred exemplary embodiment, the RF flip angle components each provide a substantially 90° ($\pi/2$ radians) nutation flip angle and have a relative RF phase difference of substantially 180° $\pi$ radians. Furthermore, according to the invention, the evolved phase difference between different species is substantially $\pi$ - and, in a particularly preferred exemplary embodiment, the chemical phase shift separated NMR species are water and fat species separated by about 3.4 ppm (parts per million) in the frequency domain.

As will be explained in more detail, the prep scan/MRI data acquisition process is repeated with differing prep scan parameters (phase cycling of the relative RF phase used for at least one of the binomial pulse components) followed by combining of the plural sets of acquired MRI data to provide a more uniform signal across a spectrum of off-resonance NMR signals and thus a more uniform final image (e.g., by using a sum of squares (SSQ) or maximum intensity projection (MIP) or the like data combination processes).

A conventional MRI data acquisition sequence following the special preparatory NMR sequence may include for example, a fast spin echo (FSE), a single shot fast spin echo (SSFSE), a fast asymmetric spin echo (FASE), a variable flip angle (VFA) or a steady-state free precession (SSFP) data acquisition sequence - or any other desired MRI data acquisition sequence.

The exemplary embodiments create Dixon images using a unique prepulse. The exemplary embodiments can create Dixon water/fat opposed-phase (WFOP) fat-suppressed images. In a more sophisticated scheme, the prepulse can be used to generate fat/water separation images (similar to LAVA (Liver Acquisition with Volume Acquisition), IDEAL (iterative Decomposition of water and fat with Echo Asymmetry and Least squares estimation), etc.).

The WFOP and fat/water separation images can be acquired without imposing restrictions on the subsequent main MRI data acquisition pulse sequence timing including TE/TR, inter-echo spacing, or readout bandwidth. For these reasons, the primary application of the exemplary embodiments is likely to generate fat/water separation images using aggressive readout methods including short echo space FSE (FASE or VFA) or SSFP imaging.

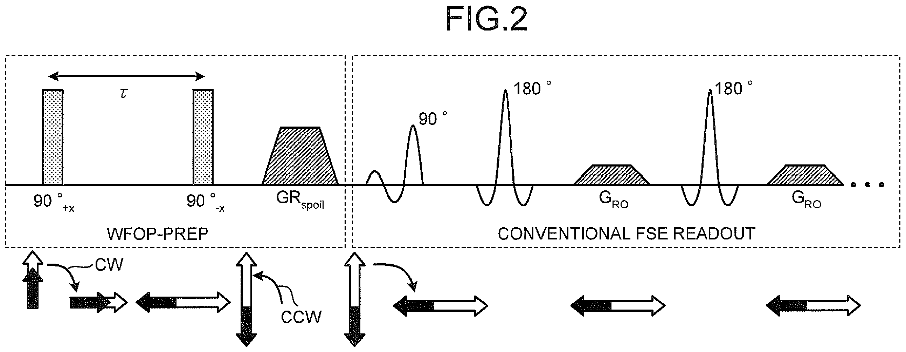

The exemplary WFOP-prep sequence of FIG. 2 consists of a binomial RF nutation pulse having two 90° flip pulse components. The 90° RF components can be made non-selective or slice/slab-selective. The relative transmit phase of the RF pulse components is a design parameter which affects water/fat phase evolution. In the simplest exemplary scheme, the transmit phase of the RF flip components is opposite (+x, -x, e.g., +90°, -90°).

The exemplary WFOP-prep sequence of FIG. 2 is attached to a conventional FSE readout main MRI data acquisition sequence. The inter-pulse phase evolution time $\tau$ is preferably 1.15 ms at 3 T for water/fat separation. The evolution of fat (solid arrow) and water (open arrow) isochromats during the sequence is displayed with schematic 90° clockwise (CW) and 90° counterclockwise (CCW) flip angles depicted corresponding to relative RF component phasing of 180° (e.g., x = $\pi/2$). Although the RF pulses can be made slice- or slab-selective, in this diagram they are illustrated as non-selective pulses.

The exemplary RF components are separated by an out-of-phase time period (1.15 ms at 3 T for water/fat separation). The first pulse nutates longitudinal magnetization into the transverse plane. The relative phase angle between fat and water evolves during the inter-pulse period $\tau$. After a time $\tau$ equal to 1.15 ms for opposed water/fat phase separation, the transverse magnetization is reversely nutated back into the longitudinal axis, thereby storing the evolved phase angle between fat and water. The opposed-phase longitudinal magnetization is then read using any conventional MRI readout sequence, without the need for pulse/echo shifting or readout adjustment. In the case of FSE, the refocus pulse(s) of the echo train realign the transverse magnetization with its original relative phase (fat/water opposed) at each echo.

The exemplary WFOP-prep sequence uses a binomial (1:1) RF composite pulse, which is somewhat similar to prior use for spectral-selective excitation. However, the new exemplary WFOP-prep sequence is designed to leave both vectors (fat and water) in the longitudinal axis. In conventional binomial spectral-selective excitation, one species vector is put into the transverse plane (generally water) and the other species vector is left in the longitudinal axis (generally fat). In this way, WFOP-prep resembles a binomial spectral-selective inversion pulse as opposed to a binomial spectral-selective excitation pulse. With WFOP-prep, one vector is selectively inverted as opposed to one vector being selectively excited.

The WFOP-prep prepulse can be used to generate simple Dixon images. The most common use for this exemplary embodiment is simple fat suppression using WFOP-prep with $\tau = \pi$ to provide some fat cancellation. However, multiple WFOP-prep images with additional, different phase evolution periods ($\tau$) can be combined to generate fat/water separation images.

Since $\tau$ is a free parameter, WFOP-prep can be used as part of a multi-point Dixon method fat/water separation. With WFOP-prep, multiple image sets can be acquired with different $\tau$. Since the relative evolved phase angle is developed as part of the prep sequence, and not the MRI acquisition and readout sequence, the data at each $\tau$ time is acquired with identical contributions from other sequence-dependent sources of phase (i.e., eddy currents) and stimulated echo signal. This uniformity in all data sets can help simplify analysis of the resulting multi-point Dixon data.

The brain of a male volunteer was scanned on a 3 T whole-body research system under IRB approval Axial partial-Fourier 3D FSE with and without WFOP-preparation were acquired with the following parameters: TE/TR = 80/3000 ms, echo space = 5.0 ms, 2 shots, ETL = 80, matrix = 256 × 256, partial-Fourier factor = 5/8, FOV = 25 × 25 cm, sixteen 3 mm thick slices and readout BW = 651 Hz/pixel. For comparison, analogous images with CHESS (chemical shift selective) fat saturation were acquired.

To measure the fat suppression effect, ROIs (regions of interest) were placed at four locations in the fat region under the scalp, four locations in white matter (WM), and four locations in gray matter (GM).

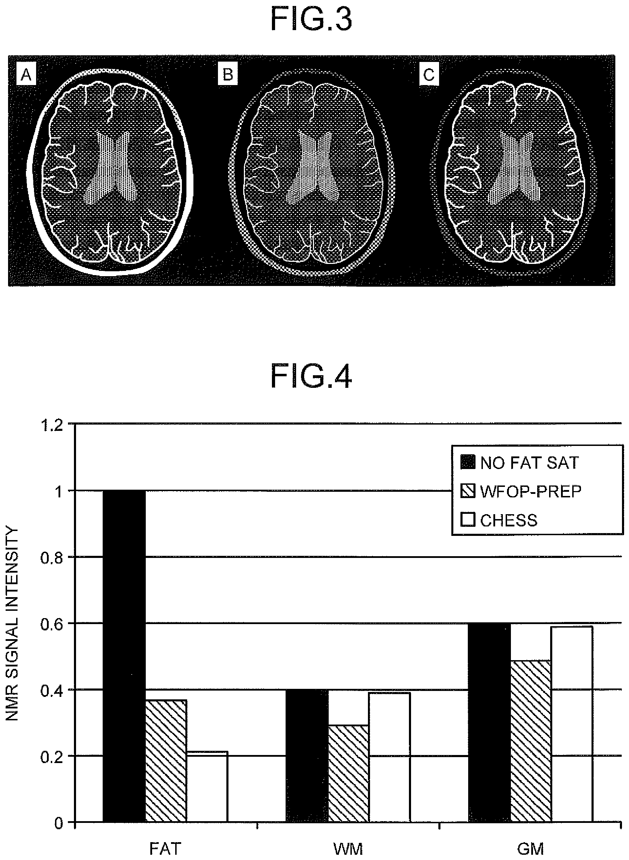

Representative images without fat saturation (A) and with WFOP-prep (B) or CHESS fat saturation (C) are displayed in FIG. 3. The WFOP-prep (B) removed most of the fat signal without deleteriously affecting water signal.

While this simple single image feasibility study image 3(B) is not as good as the CHESS image 3(C), multiple images combined will improve that and, unlike with CHESS, one can obtain true water-only and fat-only images using the exemplary WFOP-prep scan.

The WFOP-prep sequence enabled Dixon image acquisition with a conventional short echo space FSE readout. The choice of echo spacing, readout bandwidth and other sequence parameters can be made independently of the $\tau$ time. This freedom allows FSE-based Dixon methods to be used in conjunction with applications with short echo-spacing, such as single-shot FSE.

When used as a part of a single acquisition, the WFOP-prep sequence is vulnerable to signal loss as evidenced by the 20-25% decrease in WM and GM signals shown in FIG. 4. FIG. 4 shows signal comparison of non-fatsat, WFOP-prep and CHESS images. Data were normalized to the fat signal in the non-fat sat image. The fat signals of WFOP and CHESS were decreased by 63% and 79%, respectively. WFOP-prep had 25% and 19% decreases in WM and GM signals, respectively. The CHESS data had negligible decrease in water signal. Signal loss can come from an imperfect 90° due to $\Delta B_1$ RF field inhomogeneity and phase accrual caused by background $\Delta B_0$ static field inhomogeneity during the $\tau$ free evolution time. For these reasons, WFOP-prep in a single acquisition may be applicable only in regions with adequate $B_0$ and $B_1$ homogeneity. The signal loss due to background phase accrual is sinusoidal. It can be relieved as described below.

The relative transmit RF phase of the WFOP-prep sequence RF components can be alternated to "phase-cycle" the WFOP-prep data. This phase-cycling is similar in concept and design to SSFP (i.e., CISS (Constructive Interference Steady-State)).

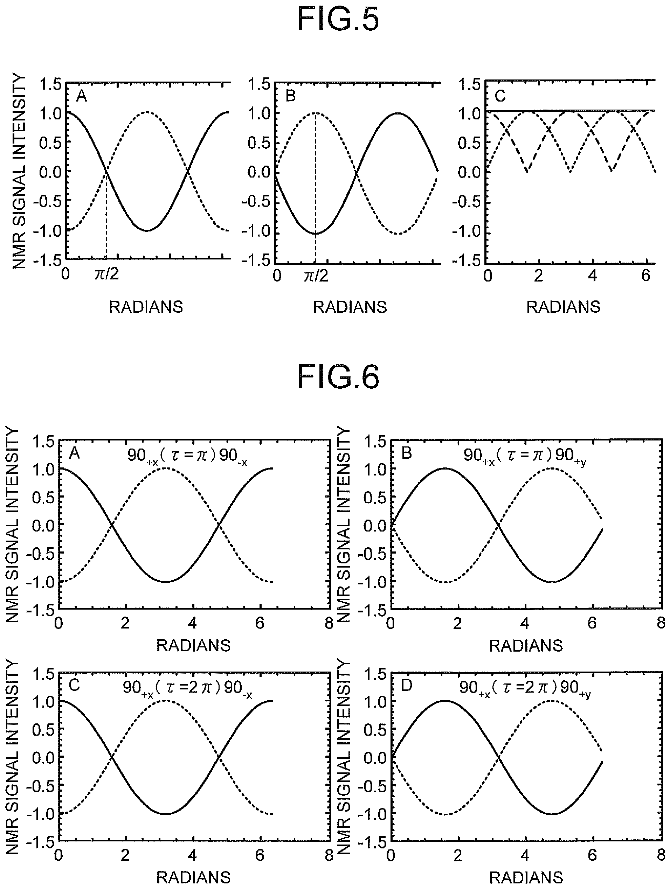

For example, a first WFOP-prep acquisition can use: $90_{+x}$ - ($\pi$ time delay) - $90_{-x}$. At $\pi/2$ off-resonance (equivalent to 110 Hz at 3 T), the signals from both fat and water disappear as seen in FIG. 5A. However, an additional WFOP-prep sequence can be acquired using $90_{+x}$ - ($\pi$ time delay) - $90_{+y}$. In this second acquisition, on-resonant spins (fat and water) will also disappear, but the $\pi/2$ off-resonant spins will be at full signal as shown in FIG. 5B.

The images from each of these data sets can be combined (using MIP, SSQ, etc.) to create a final image with a more uniform signal across the spectrum of off-resonances as depicted in FIG. 5C where the dashed line represents squared data from FIG. 5B, the dotted line represents squared data from FIG. 5A, and the solid line represents the combination of the dotted and dashed lines. In this way, the final image will closely resemble a CISS image, where two SSFP images are combined to create a more uniform final image (i.e., with full signal strength across a spectrum of off-resonance nuclei).

FIG. 6 also depicts another example of sinusoidal signal modulation which causes signal loss at zero-crossings. As explained, to compensate for this signal loss, the relative transmit phase of the RF pulses can be adjusted. For example, a transmit phase scheme of $90_{+x}$ - $90_{+y}$ (FIGS. 6B and 6D) produces peaks where the $90_{+x}$ - $90_{-x}$ scheme (FIGS. 6A and 6C) produces troughs. Thus, a shift of $\pi/2$ in RF transmit phase is equivalent to a $\pi/2$ shift in the sinusoidal response.

In a simple Dixon opposed-phase image acquisition, only a single image must be acquired (e.g., using the scheme presented in FIG. 6A). However, due to this signal modulation, an additional phase cycled image can be acquired (using the scheme in FIG. 6B) and the resulting data combined (e.g., using SSQ, MIP, etc.) to create a final Dixon opposed-phase image substantially without signal loss.

For fat/water separation, it is necessary to acquire multiple images at different $\pi$ phase separation times. For any Dixon-based method, including WFOP-prep, the minimum number of individual images is two. For example, these two images acquired with $\tau = \pi$ and $\tau = 2\pi$, correspond to FIGS. 6A and 6C. However, due to the sinusoidal signal modulation, acquisition of additional images is performed. Most importantly, the transmit phase cycled $\tau = \pi$ image (corresponding to FIG. 6B) is acquired. For additional robustness, the transmit phase cycled $\tau = 2\pi$ image (corresponding to FIG. 6D) can also be acquired.

In FIG. 6: (A) Water (solid line) and fat (dotted line) signals using $\tau = \pi$ (1.15 ms) evolution period across $\pi$ spectrum of off-resonance for $90_{+x}$- $90_{-x}$ scheme where, at $\pi/2$ off-resonance, the signals disappear; (B) with transmit phase scheme ($90_{+x}$-$90_{+y}$) and $\tau = \pi$, the signal responses of fat and water are shifted and now, at $\pi/2$ off-resonance, fat and water are at full signals (still with opposite phases); (C) transmit phase scheme ($90_{+x}$- $90_{-x}$) and $\tau = 2\pi$, fat and water are aligned, but continue to cycle due to off-resonance; and (D) transmit phase scheme ($90_{+x}$-$90_{+y}$) and $\tau = 2\pi$ where, again, fat and water are aligned.

The images (hereby referred to as A, B, C and D corresponding to their $\tau$ times and transmit phase schemes in FIG. 6) can be input into a linear system to solve for the water and fat components. The simplified equations for the data sets can be written as below using $\rho_w$, for water spin density, $\rho_f$ for fat spin density, and $\phi$ for background phase (equivalent to $\Delta B_0$).

$$\begin{aligned}
A &= \left(\rho_w - \rho_f\right) \cdot \cos\phi \\
B &= \left(\rho_w - \rho_f\right) \cdot \sin\phi \\
C &= \left(\rho_w + \rho_f\right) \cdot \cos 2\phi \\
D &= \left(\rho_w + \rho_f\right) \cdot \sin 2\phi
\end{aligned}$$ (Equations 1)

The input data for A, B, C and D only needs to be signed magnitude data, not complex data. With this information, a simple phase map can be derived using B and A (alternatively, D and C). It is noted that phase unwrapping is not necessary. Since this does not operate on complex image data, only signed magnitude, the absolute phase is irrelevant, and only the relative phase is relevant. Alternatively, an external phase map (e.g., from shimming data acquisition) can be used or the phase map can be solved iteratively (like in IDEAL).

$$\tan^{-1}\left(\frac{C}{A}\right) = \tan^{-1}\left(\frac{\left(\rho_w - \rho_f\right) \cdot \sin\phi}{\left(\rho_w - \rho_f\right) \cdot \cos\phi}\right) = \tan^{-1}\left(\frac{\sin\phi}{\cos\phi}\right) = \phi \qquad \text{(Equation 2)}$$

Using the solution for $\phi$, Equations 1 can be rewritten in matrix format as shown below (Equations 3) and solved using linear algebra methods to find $\rho_w$ and $\rho_f$. In this example, singular value decomposition (SVD) has been chosen as the method to invert the $\phi$ matrix, but there are other available numerical methods as will be appreciated.

$$\begin{bmatrix} A \\ C \\ B \\ D \end{bmatrix} = \begin{bmatrix} \cos\phi & -\cos\phi \\ \cos 2\phi & \cos 2\phi \\ \sin\phi & -\sin\phi \\ \sin 2\phi & \sin 2\phi \end{bmatrix} \begin{bmatrix} \rho_w \\ \rho_f \end{bmatrix}$$

$$\begin{bmatrix} \rho_w \\ \rho_f \end{bmatrix} = \begin{bmatrix} \cos\phi & -\cos\phi \\ \cos 2\phi & \cos 2\phi \\ \sin\phi & -\sin\phi \\ \sin 2\phi & \sin 2\phi \end{bmatrix}^{-1} \begin{bmatrix} A \\ C \\ B \\ D \end{bmatrix}$$ (Equations 3)

The above equations have been constructed for a four input image case. Of course, more images can be acquired with different $\tau$ and/or relative transmit phase and added to the matrix. The minimum number of images is two. The ideal number of input images is a function of the amount of background phase and SNR (signal-to-noise ratio).

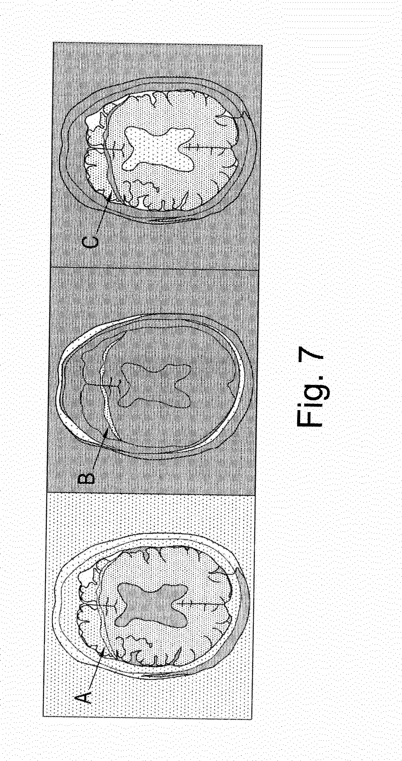

Using the above solution will produce an estimate of $\rho_w$ and $\rho_f$ at each pixel location. However, the result is sensitive to the polarity of the input signed magnitude reconstruction. At the junction between positive and negative polarities, the assignment between fat and water can be incorrect for some pixels. An example of this problem is illustrated in FIG. 7. Here, an example of incorrect fat/water assignment is shown near the polarity crossing (arrows): (A) input signed magnitude image where gray = 0, bright = first polarity, dark = opposite polarity, and using this input data produces fat (B) and water (C) images with incorrect pixel assignment near the polarity crossing shown in (A).

One method to alleviate this sensitivity is to generate a high quality, robust, signed magnitude reconstruction, for which there are many methods published in the literature. Most methods are dependent on good SNR of the input data and/or require a well-behaved phase map with slowly spatially varying phases.

However, for this exemplary embodiment, there is no need to be concerned with the signed magnitude data itself, but rather the final water and fat separation images. Therefore, a simple and robust solution can be effected to remove sensitivity to polarity in the signed magnitude images.

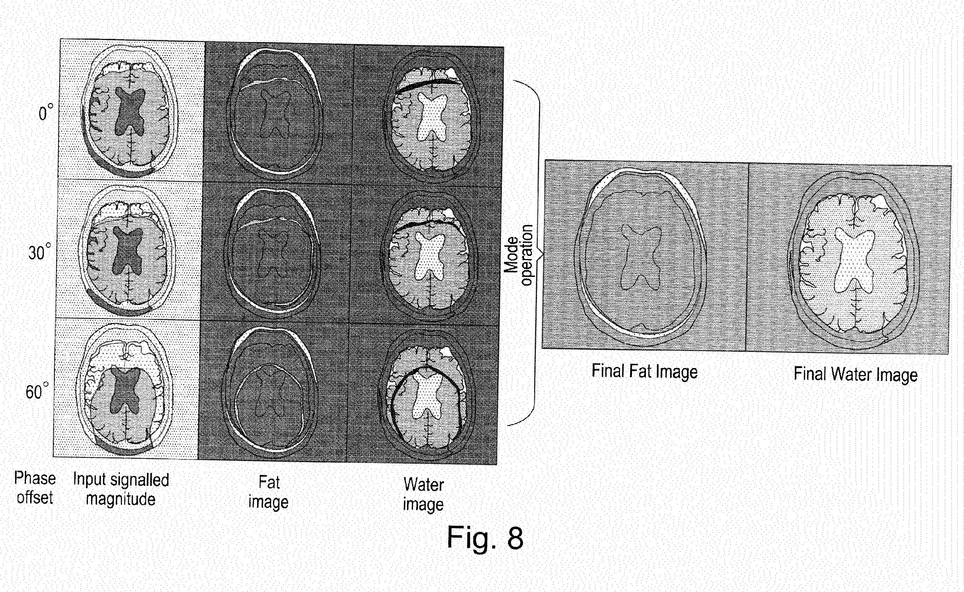

For example, fat/water separation can be effected using phase offsets. For each input image data set, the simple magnitude and phase ($\theta$) images are generated as shown in FIG. 8. Using the phase image, a positive polarity is assigned for each pixel with $\theta \geq 0$ and negative polarity to each pixel with $\theta < 0$. The fat/water separation is performed using Equations 2 and 3. The entire process is repeated N times, each time adding a fixed phase offset to the input phase image. For example, the process could be repeated six times with phase offsets of 0, 30, 60, 90, 120 and 150 degrees. The number of phase offsets and their increments is essentially arbitrary. Fat and water images are generated for each phase offset (as shown in FIG. 8). The effect of the phase offset is to shift the position of the positive/negative polarity junction in the input signed magnitude data. The fat/water output values of the SVD solution will remain the same for all pixels, except those near the junction. The most common value (mode) of the total set is assigned as the value in the final fat/water image. An example implementation of the algorithm is displayed in FIG. 8 where, in this example, three phase offsets are used. The output fat/water images of each phase offset are combined using a mode operation to create the final fat/water images.

The relative RF transmit phase of the RF components in the WFOP-prep prepulse can be adjusted to generate different fat/water sinusoidal responses. Multiple images at different relative RF transmit phases can be combined to create a Dixon image with a robust off-resonance response.

It is not strictly necessary that the RF components have different transmit phases. If they have the same transmit phase (i.e., +x, +x), it would produce negative water and positive fat, but would still work. Thus, the components are independently phased, which encompasses the ability to be different, but not necessarily so.

Alternative embodiments discussed in the just prior two paragraphs can be combined to produce fat/water separation images with a robust off-resonance response.

The number of RF pulses and/or components in the WFOP-prep module can be modified. For example, a 1:3:3:1 composite binomial RF pulse can be used. Different binomial schemes (1:1, 1:2:1, 1:3:3:1, etc.) will yield different off-resonance response shapes. In general, the more component pulses in the composite binomial pulse, the closer the response will come to resemble a boxcar shape. The 1:1 example offers the simplest response shape (sinusoidal) and thus is a presently preferred choice.

WFOP-prep enables Dixon imaging without restrictions on pulse sequence timing (TE/TR, inter-echo spacing, readout bandwidth, etc.). Therefore, it can be used in conjunction with aggressive readout methods (i.e., short echo-space FSE and short TE SSFP) which have been previously difficult or impossible to use for Dixon imaging.

The WFOP-prep sequence is insensitive to time-dependent sources of background phase including eddy currents, which confound conventional Dixon fat/water separation methods (IDEAL).

The WFOP-prep can use fast, straightforward processing with simple magnitude and phase image input. Unlike other methods, there is no need for sophisticated phase unwrapping, background phase removal, or homodyne processing.

Like all Dixon-based methods, WFOP-prep requires a minimum of two input images for full separation Dixon water/fat images. However, the input images cannot be acquired in the same main data acquisition sequence, as they could be with a multi-echo gradient echo readout method. Therefore, WFOP-prep may not be a good substitute for multi-echo gradient echo imaging (i.e., LAVA).

Furthermore, WFOP-prep potentially suffers from signal loss due to off-resonance. To mitigate this signal loss, additional images with RF transmit phase cycling are needed.

WFOP-prep requires flip-down and flip-up RF pulses to transition the magnetization to and from the transverse plane (where phase evolution occurs). Therefore, WFOP-prep can be sensitive to $B_1$ RF inhomogeneity. If the initial flip down RF pulse with flip angle $\alpha$ is not truly 90°, some magnetization will remain in the longitudinal axis ($\cos \alpha$). After the phase evolution period, the transverse magnetization may be flipped back up into the longitudinal axis with the same flip angle $\alpha$. The final longitudinal magnetization then will be a combination of phase-evolved magnetization (modulated by a factor of $\sin^2\alpha$) and un-phase-evolved (modulated by a factor of $\cos^2\alpha$). The mixing of evolved and un-evolved magnetization disrupts fat/water separation processing. For pixels with nearly 100% fat or water ($\rho_w > 0.9$ or $\rho_f > 0.9$), large deviations ($\alpha < 70°$ or $\alpha > 110°$, roughly 20% $\Delta B_1$) may cause incorrect assignments of fat/water pixel values.

While certain embodiments of the inventions have been described, these embodiments have been presented by way of example only, and are not intended to limit the scope of the inventions. Indeed, the novel methods and systems described herein may be embodied in a variety of other forms. The accompanying claims are intended to cover such forms or modifications as would fall within their scope.

## Claims

1. A magnetic resonance imaging system comprising:

a sequence executing unit (30) configured to execute a prep applying portion including a first prepulse for exciting magnetization vectors of a plurality of NMR species having different resonant frequencies due to chemical shift as transverse magnetizations and a second prepulse for nutating back the transverse magnetizations of a subject NMR species out of the plurality of NMR species into longitudinal magnetization, the second prepulse being applied after a phase evolution time by which a predetermined phase difference is evolved between the subject NMR species and the other NMR species of the plurality of NMR species after the first prepulse, and execute a data acquisition sequence after the prep applying portion, wherein the first and second pre-pulses are the RF components of a binomial pulse spaced by the phase evolution time and are followed by at least one magnetic gradient spoiler pulse before the data acquisition sequence, and wherein the predetermined phase difference is substantially equal to $\pi$; and
an image generating unit (42) configured to generate a separation image of the subject NMR species based on the data acquired by the data acquisition sequence;
**characterised in that** the sequence executing unit (30) is configured to execute the prep applying portion and the data acquisition sequence repeatedly while cycling a transmit phase of at least one of the first and second prepulse in such a manner that combining the plural sets of acquired data provides a more uniform signal across a spectrum of off-resonance NMR signals, and
the image generating unit (42) is configured to generate the separation image by combining data acquired by repeatedly executing the data acquisition sequence.

2. The magnetic resonance imaging system according to claim 1, wherein the sum of said RF components provides a substantially 180° nutation flip angle.

3. The magnetic resonance imaging system according to claim 1 or 2, wherein the plurality of NMR species include water and fat.

4. The magnetic resonance imaging system according to any preceding claim, wherein the image generating unit (42) is configured to generate the separation image by using at least one of sum of squares (SSQ) and maximum intensity projection (MIP) calculations.

5. The magnetic resonance imaging system according to any one of claims 1 to 4, wherein the data acquisition sequence includes at least one of:

a fast spin echo (FSE) MRI data acquisition sequence;
a single-shot fast spin echo MRI data acquisition sequence;
a fast asymmetric spin echo (FASE) MRI data acquisition sequence;
a variable flip angle (VFA) MRI data acquisition sequence; and
a steady-state free precession (SSFP) MRI data acquisition sequence.

6. A magnetic resonance imaging process comprising:

executing a prep applying portion including a first prepulse for exciting magnetization vectors of a plurality of NMR species having different resonant frequencies due to chemical shift as transverse magnetizations and a second prepulse for nutating back the transverse magnetizations of a subject NMR species of the plurality of NMR species into longitudinal magnetization, the second prepulse being applied after a phase evolution time by which a predetermined phase difference is evolved between the subject NMR species and the other NMR species of the plurality of NMR species after the first prepulse, wherein the first and second prepulses are the RF components of a binomial pulse separated by the phase evolution time and are followed by at least one magnetic gradient spoiler pulse before the data acquisition sequence, and wherein the predetermined phase difference is substantially equal to $\pi$;

executing a data acquisition sequence after the prep applying portion; and

generating a separation image of the subject NMR species based on the data acquired by the data acquisition sequence;

**CHARACTERISED IN THAT**:

the prep applying portion and the data acquisition sequence are repeatedly executed while cycling a transmit phase of at least one of the first and second prepulse included in the prep applying portion in such a manner that combining the plural sets of acquired data provides a more uniform signal across a spectrum of off-resonance NMR signals, and

the separation image is generated by combining data acquired by repeatedly executing the data acquisition sequence.

**Patentansprüche**

1.  Magnetresonanzbildgebungssystem, umfassend:

    eine sequenzausführende Einheit (30), konfiguriert zum Ausführen eines Zubereitungsauftragsabschnitts, umfassend einen ersten Vorimpuls zum Erregen der Magnetisierungsvektoren einer Mehrzahl von MKR-Spezies, welche unterschiedliche Resonanzfrequenzen aufweisen, wegen einer chemischen Verschiebung, als Quermagnetisierungen, und einen zweiten Vorimpuls zur Rück-Nutation der Quermagnetisierungen einer gegenständigen MKR Spezies aus der Mehrzahl von MKR Spezies in eine Längsmagnetisierung, wobei der zweite Vorimpuls nach einer Phasenevolutionszeit angewendet wird, durch welchen ein vorbestimmter Phasenunterschied zwischen der gegenständlichen MKR Spezies und den anderen MKR Spezies der Mehrzahl von MKR Spezies nach dem ersten Vorimpuls evolviert und zum Ausführen einer Datenerfassungssequenz nach dem Zubereitungsauftragsabschnitt, wobei der erste und der zweite Vorimpuls die RF-Komponenten eines binomischen Impulses sind, welche durch die Phasenevolutionszeit beabstandet sind und durch mindestens einen magnetischen Gradientenspoiler-Impuls gefolgt sind, vor der Datenerfassungssequenz, und wobei der vorbestimmte Phasenunterschied im Wesentlichen gleich $\pi$ ist; und

    eine Bilderzeugungseinheit (42), welche konfiguriert ist, um ein Auszugsbild der gegenständlichen MKR-Spezies zu erzeugen, basierend auf den Daten, welche durch die Datenerfassungssequenz erfasst wurden;

    **dadurch gekennzeichnet, dass** die sequenzausführende Einheit (30) konfiguriert ist, um den Zubereitungsauftragsabschnitt und die Datenerfassungssequenz wiederholt auszuführen, während eine Übertragungsphase von mindestens einem des ersten und zweiten Vorimpulses zyklisch wiederholt wird, sodass das Kombinieren der mehreren Sätze von erfassten Daten ein gleichmäßigeres Signal über ein Spektrum von außer-Resonanz-MKR-Signalen bereitstellt, und

    die Bilderzeugungseinheit (42) konfiguriert ist, um das Auszugsbild zu erzeugen, indem Daten kombiniert werden, welche durch wiederholtes Ausführen der Datenerfassungssequenz erfasst wurden.

2.  Magnetresonanzbildgebungssystem nach Anspruch 1, wobei die Summe der RF-Komponenten einen Nutations-Kippwinkel von im Wesentlichen 180° bereitstellt.

3.  Magnetresonanzbildgebungssystem nach Anspruch 1 oder 2, wobei die Mehrzahl von MKR-Spezies Wasser und Fett umfasst.

4.  Magnetresonanzbildgebungssystem nach einem der vorhergehenden Ansprüche, wobei die Bilderzeugungseinheit (42) konfiguriert ist, um das Auszugsbild unter Verwendung mindestens einer von der Quadratsummen-(SSQ)- und der maximalen Intensitätsprojektion-(MIP)-Berechnung zu erzeugen.

5.  Magnetresonanzbildgebungssystem nach einem der Ansprüche 1 bis 4, wobei die Datenerfassungssequenz mindestens eine der folgenden umfasst:

    eine Fast-Spin-Echo-(FSE)-MRT-Datenerfassungssequenz;
    eine Single-Shot-Fast-Spin-Echo-MRT-Datenerfassungssequenz;
    eine Fast-Asymmetric-Spin-Echo-(FASE)-MRT-Datenerfassungssequenz;
    eine variable Kippwinkel-(VFA)-MRT-Datenerfassungssequenz; und
    eine Steady-State-Free-Precession-(SSFP)-MRT-Datenerfassungssequenz.

6. Magnetresonanzbildgebungsverfahren, umfassend:

Ausführen eines Zubereitungsauftragsabschnitts, umfassend einen ersten Vorimpuls zum Erregen der Magnetisierungsvektoren einer Mehrzahl von MKR-Spezies, welche unterschiedliche Resonanzfrequenzen aufweisen, wegen einer chemischen Verschiebung, als Quermagnetisierungen, und einen zweiten Vorimpuls zur Rück-Nutation der Quermagnetisierungen einer gegenständigen MKR Spezies aus der Mehrzahl von MKR Spezies in eine Längsmagnetisierung, wobei der zweite Vorimpuls nach einer Phasenevolutionszeit angewendet wird, durch welchen ein vorbestimmter Phasenunterschied zwischen der gegenständlichen MKR Spezies und den anderen MKR Spezies der Mehrzahl von MKR Spezies nach dem ersten Vorimpuls evolviert, wobei der erste und der zweite Vorimpuls die RF-Komponenten eines binomischen Impulses sind, welche durch die Phasenevolutionszeit beabstandet sind und durch mindestens einen magnetischen Gradientenspoiler-Impuls gefolgt sind, vor der Datenerfassungssequenz, und wobei der vorbestimmte Phasenunterschied im Wesentlichen gleich $\pi$ ist;
Ausführen einer Datenerfassungssequenz nach dem Zubereitungsauftragsabschnitt; und
Erzeugen eines Auszugsbilds der gegenständlichen MKR-Spezies, basierend auf den Daten, welche durch die Datenerfassungssequenz erfasst wurden;
**dadurch gekennzeichnet, dass**:

der Zubereitungsauftragsabschnitt und die Datenerfassungssequenz wiederholt ausgeführt werden, während eine Übertragungsphase von mindestens einem des ersten und zweiten Vorimpulses, die in dem Zubereitungsauftragsabschnitt enthalten sind, zyklisch wiederholt wird, sodass das Kombinieren der mehreren Sätze von erfassten Daten ein gleichmäßigeres Signal über ein Spektrum von außer-Resonanz-MKR-Signalen bereitstellt, und
das Auszugsbild erzeugt wird indem Daten kombiniert werden, welche durch wiederholtes Ausführen der Datenerfassungssequenz erfasst wurden.

**Revendications**

1. Système d'imagerie à résonance magnétique, comprenant :

une unité d'exécution de séquence (30) configurée pour exécuter une partie d'application de préparation incluant une première pré-impulsion permettant d'exciter des vecteurs de magnétisation d'une pluralité d'espèces de RMN présentant des fréquences de résonance différentes du fait du déplacement chimique comme magnétisations transversales et une seconde pré-impulsion pour contre-nutation des magnétisations transversales d'une espèce de RMN sujet parmi la pluralité d'espèces de RMN dans une magnétisation longitudinale, la seconde pré-impulsion étant appliquée après un temps d'évolution de phase par lequel une différence de phase prédéterminée évolue entre l'espèce de RMN sujet et les autres espèces de RMN de la pluralité d'espèces de RMN après la première pré-impulsion, et exécuter une séquence d'acquisition de données après la partie d'application de préparation, dans lequel la première et la seconde pré-impulsion sont les composants RF d'une impulsion binomiale espacée par le temps d'évolution de phase et sont suivies par au moins une impulsion de spoiler à gradient magnétique avant la séquence d'acquisition de données et dans lequel la différence de phase prédéterminée est sensiblement égale à $\pi$, et
une unité de génération d'image (42) configurée pour générer une image de séparation de l'espèce de RMN sujet sur la base des données acquises par la séquence d'acquisition de données ;
**caractérisé en ce que** l'unité d'exécution de séquence (30) est configurée pour exécuter la partie d'application de préparation et la séquence d'acquisition de données de manière répétée tout en effectuant un cycle d'une phase de transmission d'au moins une de la première et de la seconde pré-impulsion, de telle sorte que la combinaison des plusieurs ensembles de données acquises fournit un signal plus uniforme sur un spectre de signaux RMN hors résonance, et
l'unité de génération d'image (42) est configurée pour générer l'image de séparation en combinant des données acquises en exécutant de manière répétée la séquence d'acquisition de données.

2. Système d'imagerie à résonance magnétique selon la revendication 1, dans lequel la somme desdits composants RF fournit un angle de bascule de nutation sensiblement à 180°.

3. Système d'imagerie à résonance magnétique selon la revendication 1 ou 2, dans lequel la pluralité d'espèces de RMN inclut l'eau et la graisse.

4. Système d'imagerie à résonance magnétique selon l'une quelconque des revendications précédentes, dans lequel l'unité de génération d'image (42) est configurée pour générer l'image de séparation en utilisant au moins un des calculs de la somme des carrés (SSQ) et de la projection d'intensité maximale (MIP).

5. Système d'imagerie à résonance magnétique selon l'une quelconque des revendications 1 à 4, dans lequel la séquence d'acquisition de données inclut au moins un de :

une séquence d'acquisition de données IRM à écho de spin rapide (FSE) ;
une séquence d'acquisition de données IRM à écho de spin rapide unique ;
une séquence d'acquisition de données IRM à écho de spin asymétrique rapide (FASE) ;
une séquence d'acquisition de données IRM à angle de bascule variable (VFA) ; et
une séquence d'acquisition de données IRM à précession libre en régime stationnaire (SSFP).

6. Procédé d'imagerie à résonance magnétique comprenant :

l'exécution d'une partie d'application de préparation incluant une première pré-impulsion permettant d'exciter des vecteurs de magnétisation d'une pluralité d'espèces de RMN présentant des fréquences de résonance différentes du fait du déplacement chimique comme magnétisations transversales et une seconde pré-impulsion pour contre-nutation des magnétisations transversales d'une espèce de RMN sujet parmi la pluralité d'espèces de RMN dans une magnétisation longitudinale, la seconde pré-impulsion étant appliquée après un temps d'évolution de phase par lequel une différence de phase prédéterminée évolue entre l'espèce de RMN sujet et les autres espèces de RMN de la pluralité d'espèces de RMN après la première pré-impulsion, dans lequel la première et la seconde pré-impulsion sont les composants RF d'une impulsion binomiale espacée par le temps d'évolution de phase et sont suivies par au moins une impulsion de spoiler à gradient magnétique avant la séquence d'acquisition de données et dans lequel la différence de phase prédéterminée est sensiblement égale à $\pi$ ;
l'exécution d'une séquence d'acquisition de données après la partie d'application de préparation ; et
la génération d'une image de séparation de l'espèce RMN sujet sur la base des données acquises par la séquence d'acquisition de données ;
**caractérisé en ce que** :
la partie d'application de préparation et la séquence d'acquisition de données sont exécutées de manière répétée tout en effectuant un cycle d'une phase de transmission d'au moins une de la première et de la seconde pré-impulsion incluses dans la partie d'application de préparation de sorte que la combinaison des plusieurs ensembles de données acquises fournit un signal plus uniforme sur un spectre de signaux RMN hors résonance, et
l'image de séparation est générée en combinant des données acquises en exécutant de manière répétée la séquence d'acquisition de données.

# FIG.1

FIG.2

EP 2 508 910 B1

# FIG.3

# FIG.4

# FIG.5

# FIG.6

Fig. 7

Fig. 8

**EP 2 508 910 B1**

**REFERENCES CITED IN THE DESCRIPTION**

**Patent documents cited in the description**

- JP 2010162096 A

- US 2007285090 A